(19) Europäisches Patentamt
European Patent Office
Office européen des brevets

(11) Publication number: **0 044 142**
**A1**

(12) # EUROPEAN PATENT APPLICATION

(21) Application number: 81302738.0

(22) Date of filing: 17.06.81

(51) Int. Cl.³: C 07 D 487/04
A 61 K 31/40
//(C07D487/04, 205/00, 209/00)

(30) Priority: 03.07.80 GB 8021782

(43) Date of publication of application: 20.01.82 Bulletin 82/3

(84) Designated Contracting States: BE CH DE FR GB IT LI NL

(71) Applicant: BEECHAM GROUP LIMITED
Beecham House Great West Road
Brentford, Middlesex(GB)

(72) Inventor: Corbett, David Francis
72E Holmesdale Road
Reigate Surrey(GB)

(74) Representative: Hesketh, Alan, Dr. et al,
European Patent Attorney Beecham Pharmaceuticals
Great Burgh Yew Tree Bottom Road
Epsom, Surrey, KT18 5XQ(GB)

(54) **Process for the preparation of beta-lactam antibiotics.**

(57) The present invention provides a process for the preparation of antibacterially active carbapenems of the formula:

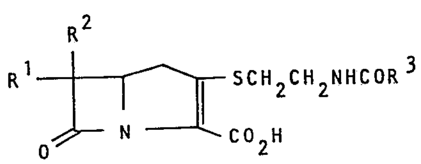

and salts and esters thereof wherein $R^1$ and $R^2$ are independently hydrogen or an organic group bonded *via* a carbon atom and $R^3$ is a group $NR^4R^5$ wherein $R^4$ optionally substituted alkyl, aryl and aralkyl, and $R^5$ is hydrogen or optionally substituted alkyl or $R^3$ is optionally substituted aryl or alkyl: which process comprises the reaction of a C−3 mercapto carbapenem with an aziridine $(CH_2)_2NCOR^3$.

EP 0 044 142 A1

# Process for the preparation of β-Lactam Antibiotics

This invention relates to a novel process for preparing β-lactam antibiotics, in particular carbapenem derivatives. The process also enables certain novel derivatives to be prepared.

European Patent Specification No. 0001628 discloses a group of synthetic antibacterial agents containing a 7-oxo-1-azabicyclo[3.2.0]hept-2-ene ring system, i.e. carbapenem derivatives. However all compounds described in that specification were racemic at C-5 and could only be prepared by a lengthy synthetic sequence. We have now discovered a process for preparing carbapenem derivatives from natural products in relatively few steps and produces carbapenem derivatives as a desirable single optical isomer at C-5.

The present invention provides a process for the preparation of compounds of the formula (I):

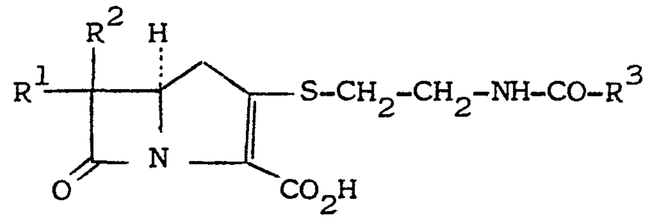

(I)

and salts and esters thereof, wherein $R^1$ and $R^2$ each represent a hydrogen atom or an organic group bonded via a carbon atom to the carbapenem ring, and $R^3$ is a group $-NR^4R^5$ wherein $R^4$ is an optionally substituted $C_{1-6}$ alkyl group or an optionally substituted aryl or heteroaryl group or optionally substituted aryl $C_{1-6}$ alkyl or heteroaryl $C_{1-6}$ alkyl group and $R^5$ is a hydrogen atom or an optionally substituted $C_{1-6}$ alkyl group, or $R^3$ is an optionally substituted aryl or heteroaryl group or an optionally substituted $C_{1-6}$ alkyl group: which process comprises the reaction of a compound of the formula (II) or a reactive derivative thereof in which reaction any reactive groups in the groups $R^1$ and $R^2$ may be protected, if desired;

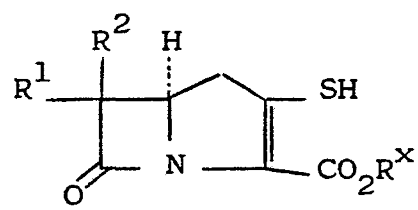

(II)

wherein $R^1$ and $R^2$ are as defined in relation to formula (I) and $R^x$ is a carboxy blocking group, with an aziridine derivative of the formula (III) in which any reactive groups in the group $R^3$ may be protected, if desired:

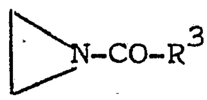

(III)

wherein $R^3$ is as defined in relation to formula (I); and thereafter optionally performing one or more of the following steps:

i) removing any protecting groups in the groups $R^1$, $R^2$ and $R^3$;

ii) removing any carboxy blocking group;

iii) converting the product into an ester, salt or acid.

Preferably in the compounds of the formula (I) either $R^1$ or $R^2$ is a hydrogen atom. In an alternative aspect both $R^1$ and $R^2$ are hydrogen atoms.

When the group $R^1$ or $R^2$ represents an organic group, it may be selected from $C_{1-10}$ alkyl, $C_{2-10}$ alkenyl, $C_{2-10}$ alkynyl, $C_{3-7}$ cycloalkyl $(C_{1-6})$ alkyl, $C_{3-7}$ cycloalkyl, aryl, aryl $(C_{1-6})$ alkyl, heterocyclyl, heterocyclyl $(C_{1-6})$ alkyl, heteroaryl and heteroaryl $(C_{1-6})$ alkyl, any of the above groups being optionally substituted. Suitably the hetero atom or hetero atoms in the above named heteroaryl and/or heterocyclyl moieties is/are selected from 1 to 4 oxygen, nitrogen or sulphur

atoms. Suitable optional substituents include $C_{1-6}$ alkyl, amino, $C_{1-6}$ alkanoylamino, mono-, di- and tri- $(C_{1-6})$ alkylamino, hydroxy, $C_{1-6}$ alkoxy, mercapto, $C_{1-6}$ alkylthio, heteroarylthio, arylthio, sulphamoyl, carbamoyl, amidino, guanidino, nitro, chloro, bromo, fluoro, carboxy and salts and esters thereof, $C_{1-6}$ alkanoyloxy, arylcarbonyl and heteroarylcarbonyl.

More suitably one of $R^1$ and $R^2$ is a hydrogen atom and the other is selected from a group of the sub-formula (i):

$$CH_3CHR^6- \qquad (i)$$

wherein $R^6$ is a hydrogen atom or a group selected from OH, $OSO_3H$ or salt or methyl or ethyl ester thereof, $OR^7$, $SR^8$, $OCO_2R^7$, $OCOR^8$ or $OCONHR^8$ wherein $R^7$ is $C_{1-4}$ alkyl, benzyl or nitrobenzyl and $R^8$ is $C_{1-4}$ alkyl, phenyl, benzyl or nitrobenzyl.

Also suitably one of $R^1$ and $R^2$ is a hydrogen atom and the other is α-hydroxypropyl or α-sulphonato-oxy-propyl or a salt or ester thereof.

Favoured values of $R^6$ include a hydroxy group and a $OSO_3H$ group or salt or methyl or ethyl ester thereof. Compounds of the formula (I) containing a sulphate group on the C-6 side chain are normally provided in the form of a pharmaceutically acceptable salt of that group.

Suitably $R^3$ is a group $NR^4R^5$ as hereinbefore defined. Suitably $R^3$ is an optionally substituted aryl or heteroaryl group or an optionally substituted $C_{1-6}$ alkyl group. Suitable substituents for $R^3$, $R^4$ and $R^5$ include those described as suitable for $R^1$ and $R^2$.

More suitably $R^4$ is $C_{1-6}$ alkyl or a phenyl or benzyl group and $R^5$ is a hydrogen atom or a $C_{1-6}$ alkyl group. More suitably also $R^3$ is an aryl or heteroaryl group or $C_{1-6}$ alkyl or $C_{1-6}$ alkyl substituted by an aryl or heteroaryl group or an amino group or by a carboxylic acid group or salt or ester thereof.

Favoured values for $R^4$ include the methyl, ethyl, phenyl and benzyl groups. Favoured values for $R^5$ include the hydrogen atom and methyl and ethyl groups. Thus particularly favoured values for the group $CONR^4R^5$ include methylaminocarbonyl, dimethylaminocarbonyl, ethylaminocarbonyl, phenylaminocarbonyl and benzylaminocarbonyl.

When $R^3$ is an aryl or heteroaryl group convenient values for $R^3$ include phenyl, furyl, pyrrolyl, thienyl, quinolyl, naphthyl, indolyl, benzofuryl, thionaphthyl, and such groups substituted by an alkyl group of 1 to 4 carbon atoms. Suitably $R^3$ is an alkyl group of 1 to 6 carbon atoms optionally substituted by an aryl group or an amino group or by a carboxylic acid group or salt or $C_{1-4}$ alkyl ester thereof; for example more suitable

values for $R^3$ include methyl, ethyl, propyl, butyl, benzyl, phenethyl, aminomethyl, aminoethyl, carboxymethyl or a salt or $C_{1-4}$ alkyl ester thereof and carboxyethyl or a salt or $C_{1-4}$ alkyl ester thereof.

Preferred values for $-COR^3$ therefore include the acetyl, propionyl, aminoacetyl, benzoyl, furoyl, thenoyl, methylaminocarbonyl and dimethylaminocarbonyl groups, of these the aminoacetyl group is particularly preferred.

The major utility of the compounds prepared by the process of this invention is as pharmaceuticals and accordingly the salts and esters of the compounds of the formula (I) are preferably pharmaceutically acceptable. The compounds of this invention both pharmaceutically acceptable and non-pharmaceutically acceptable may be used as intermediates and also as antibacterial agents or β-lactamase inhibitors in non-pharmaceutical usage such as a disinfectant or paint additive.

In a favoured aspect of this invention there is provided a process for the preparation of a class of compounds of the formula (IV):

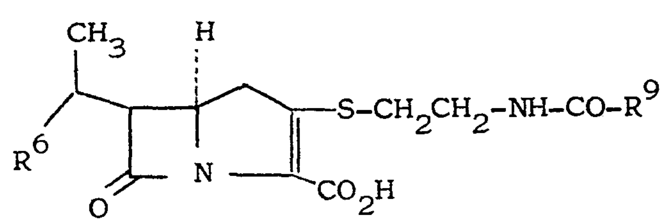

(IV)

and pharmaceutically acceptable salts and in-vivo hydrolysable esters thereof wherein $R^6$ is as hereinbefore defined, and $R^9$ is a group $-NR^{10}R^{11}$ wherein $R^{10}$ is $C_{1-6}$ alkyl, phenyl or benzyl and $R^{11}$ is hydrogen or $C_{1-6}$ alkyl, or $R^9$ is an aryl or heteroaryl group or $C_{1-6}$ alkyl optionally substituted by aryl, heteroaryl, amino or by a carboxylic acid group or salt or $C_{1-4}$ alkyl ester thereof.

Preferably in the compounds of the formula (IV) when $R^6$ is $OSO_3H$ or salt or methyl or ethyl ester thereof the C-5 and C-6 protons are cis.

Particularly apt compounds of the formula (IV) are those of the formula (V):

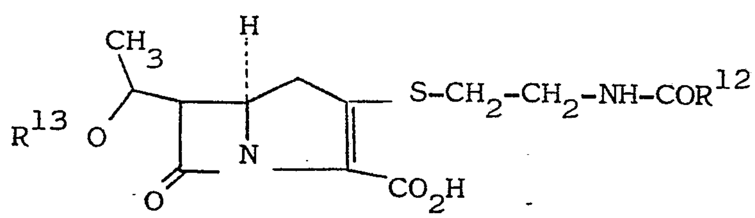

(V)

and pharmaceutically acceptable salts and in-vivo hydrolysable esters thereof wherein $R^{12}$ is an aryl or heteroaryl group or an alkyl group of 1 to 6 carbon atoms optionally substituted by an aryl or heteroaryl group or an amino group or by a carboxylic acid group or salt or $C_{1-4}$ alkyl ester thereof, and $R^{13}$ is a hydrogen atom or a pharmaceutically acceptable salt or a $SO_3H$ group.

Further particularly apt compounds of the formula (IV) are those of the formula (VI):

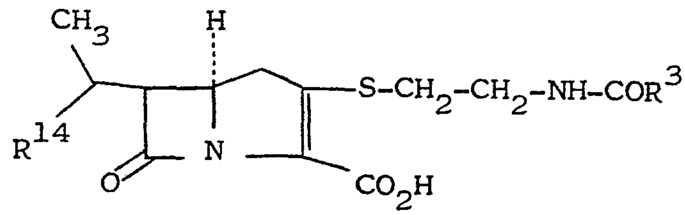

(VI)

and pharmaceutically acceptable salts and in-vivo hydrolysable esters thereof wherein $R^3$ is as defined in relation to formula (I) and $R^{14}$ is a hydrogen atom or a $SR^8$ group wherein $R^8$ is as defined hereinbefore.

The compounds of the formula (I) (wherein one of $R^1$ or $R^2$ is hydrogen and the other is not) may be in the form of cis - or trans - isomers about the β-lactam ring, that is to say they may have the 5R, 6R or 5R, 6S configuration, or they may be provided as mixtures of such isomers although this is not normally preferred. Furthermore certain compounds of the formulae (I) for example those of formula (IV) may have R or S stereochemistry at C-8 (except of course when $R^6$ is a hydrogen atom) or may be in the form of mixtures thereof. The compounds of the formula (V) wherein $R^{13}$ is a group $-SO_3H$ or a pharmaceutically acceptable salt thereof, are preferably in the 5R, 6R, 8S configuration as the intermediates are more readily available.

Preferred compounds which may be produced by the process of this invention include: Sodium (5R,6R)-3-[2-(thiophen-2-carboxamido)ethylthio]-6-[(S)-1-hydroxyethyl]-7-oxo-1-azabicyclo[3.2.0]hept-2-ene-2carboxylate, The sodium salt of p-Nitrobenzyl (5R,6R)-3-[2-(thiophen-2-carboxamido)ethylthio]-6-[(S)-1-hydroxysulphonyloxyethyl]-7-oxo-1-azabicyclo [3.2.0] hept-2-ene-2-carboxylate, The disodium salt of (5R,6R)-3-[2-(thiophen-2-carboxamido)-ethylthio]-6-[(S)-1-hydroxy sulphonyloxyethyl]-7-oxo-1-azabicyclo [3.2.0]hept-2-ene-2-carboxylic acid, (5R,6S)-3-(2-Aminoacetamidoethylthio)-6-[(S)-1-hydroxyethyl]-7-oxo-1-azabicyclo[3.2.0.]hept-2-ene-2-carboxylic acid, (5R,6R)-3-(2-Aminoacetamidoethylthio)-6-[(S)-1-hydroxyethyl]-7-oxo-1-azabicyclo[3.2.0]hept-2-ene-2-carboxylic acid.

Suitably the compounds of the formula (I) are in the form of a pharmaceutically acceptable salt, for example those of the alkali and alkaline earth metals such as the sodium, potassium, magnesium, calcium and ammonium salts. Of these the sodium and potassium salts are preferred.

It is to be realised that if a free alkylamino group ($-CH_2-NH_2$) is present in the group $R^3$ then a zwitterion of the compound of the formula (I) may be formed, said zwitterion normally being formed with the carboxylic acid function at the C-2 position.

If a carboxylic acid group is present in the group $R^3$ then optionally this may be salified by a pharmaceutically acceptable salt as hereinbefore described.

Suitable esters of the compounds of the formula (I) include those cleavable by biological methods such as enzy matic hydrolysis, in-vivo hydrolysis, and those cleavable by chemical methods such as hydrogenolysis, hydrolysis, electrolysis or photolysis.

Suitably the carboxylic acid is esterified by a group of the sub-formula (a), (b), (c), (d), (e) or (f):

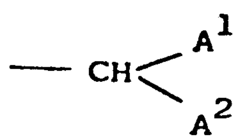
(a)

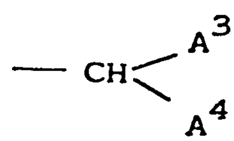
(b)

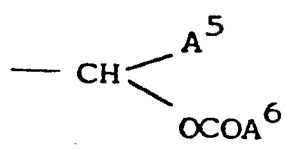
(c)

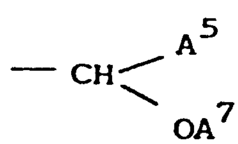
(d)

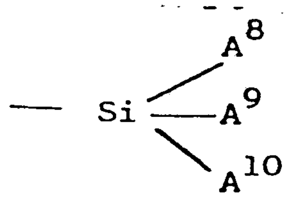
(e)

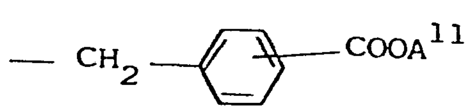
(f)

wherein $A^1$ is a hydrogen atom, $C_{1-6}$ alkanoyl or an $C_{1-5}$ alkyl group optionally substituted by $C_{1-7}$ alkoxy or $C_{1-7}$ carboxylic acyloxy, or an alkenyl or alkynyl group of up to 5 carbon atoms; $A^2$ is a hydrogen atom or a methyl group; $A^3$ is a phenyl group or a phenyl group substituted by a fluorine, chlorine or bromine atom or a nitro, $C_{1-3}$ alkyl or $C_{1-3}$ alkoxy group; $A^4$ is a hydrogen atom or a phenyl group or phenyl group substituted by a fluorine, chlorine or bromine atom or a nitro, $C_{1-3}$ alkyl or $C_{1-3}$ alkoxy group; $A^5$ is a hydrogen atom or a methyl group; $A^6$ is a $C_{1-6}$ alkyl, phenyl, phenoxy, phenyl ($C_{1-3}$) alkyl, phenyl $C_{1-3}$ alkoxy or $C_{1-4}$ alkoxy group or $A^5$ is joined to $A^6$ to form a phthalidyl, dimethylphthalidyl or dimethoxyphthalidyl group; $A^7$ is a $C_{1-4}$ alkyl, phenyl, chlorophenyl or nitrophenyl group; $A^8$ is a $C_{1-4}$ alkyl or phenyl group; $A^9$ is a $C_{1-4}$ alkyl or phenyl group; $A^{10}$ is $C_{1-4}$ alkyl; and $A^{11}$ is a $C_{1-4}$ alkyl: or $CHA^1A^2$ is a phenacyl or bromophenacyl group.

Favourably $A^1$ is a hydrogen atom or a methyl, ethyl, vinyl or ethenyl group. Favourably $A^2$ is a hydrogen atom. Favourably $A^3$ is a phenyl, p-bromophenyl, p-methoxyphenyl or p-nitrophenyl group. Favourably $A^4$ is a hydrogen atom. Favourably $A^6$ is a methyl, t-butyl or ethoxy group or is joined to $A^5$. Favourably $A^7$ is a methyl group. Favourably $A^5$ is a hydrogen atom.

Preferred groups of the sub-formula (a) include the methyl, ethyl and acetonyl groups.

Preferred groups of the sub-formula (b) include the benzyl and p-nitrobenzyl groups.

When $A^5$ is hydrogen, suitably $A^6$ is selected from methyl, ethyl, n-propyl, isopropyl, n-butyl, tert-butyl, phenyl, benzyl, methoxy, ethoxy, n-propyloxy and iso-propyloxy. Preferably $A^6$ is tert-butyl. Preferred groups of the sub-formula (c) include the acetoxymethyl, pivaloyloxymethyl, ethoxycarbonyloxymethyl, phthalidyl, α-ethoxycarbonyloxyethyl and α-acetoxyethyl groups. These esterifying groups are favoured as they tend to form in-vivo hydrolysable esters.

A preferred group of the sub-formula (d) is the methoxymethyl group.

Preferred groups of the sub-formula (e) include the trimethylsilyl, tert-butyldimethylsilyl and tert-butyldiphenylsilyl groups.

A preferred group of the sub-formula (f) is p-methoxycarbonylbenzyl.

Particularly preferred esterifying groups are the p-nitrobenzyl and phthalidyl groups.

Pharmaceutically acceptable *in-vivo* hydrolysable esters are those esters which hydrolyse in the human body to produce the parent acid or its salt. Such esters may be identified by administration to a test animal such as a rat or mouse by intravenous administration and thereafter exaining the test animal's body fluids for the presence of the compound of the formula (I) or salt.

Suitable esters of this type include those of sub-formula (c) as hereinbefore defined.

Further suitable esters include di($C_{1-6}$) alkylamino $C_{1-6}$ alkyl esters such as dimethylaminomethyl, dimethylaminoethyl, diethylaminomethyl and diethylaminoethyl.

Compounds of the formula (I) in the form of chemically cleavable esters are also of value as intermediates. Such chemically cleavable esters are preferably formed at the carboxylic acid function at the C-2 position. Suitably the esters of use as intermediates are those cleavable by catalytic hydrogenation, for example a benzyl ester optionally substituted by $C_{1-3}$ alkoxy, $C_{1-3}$ alkanoyloxy, halogen or a nitro group. Of these the p-nitrobenzyl ester is preferred.

During the process of this invention, any reactive groups in the groups $R^1$, $R^2$ and $R^3$ may be protected. For example if $R^3$ contains an amino group it may be protected by a protected amino group such as those known in the art, for example enamino groups, or p-nitrobenzyloxycarbonylamino which on hydrogenation affords an amino group. Any carbonyl group in $R^3$ may be esterified which on hydrogenation affords a carboxylic acid or salt thereof.

It must be realised that salts of the compounds of the formulae (I) formed with pharmaceutically unacceptable ions are useful as they may serve as intermediates in the preparation of pharmaceutically acceptable salts by ion-exchange, or they may be useful as intermediates in the preparation of in-vivo hydrolysable esters. An example of such a salt is the lithium salt, which may be converted into other salts such as the sodium, potassium, calcium or magnesium salt by ion-exchange.

Suitably $R^x$ in the compound of the formula (II) is an ester, suitably a cleavable ester or an in-vivo hydrolysable ester. Suitably cleavable esters of the

formula (II) for use in such a process are those that are cleavable by chemical methods. Apt esters include those cleavable by hydrogenation, for example a benzyl ester optionally substituted by $C_{1-3}$ alkoxy, $C_{1-3}$ alkanoyloxy, halogen or a nitro group. Of these the p-nitrobenzyl ester is preferred. Other apt esters for use in this process are in-vivo hydrolysable esters as hereinbefore described.

The reaction of an ester of a compound of the formula (II) with the aziridine derivative normally takes place in an inert organic solvent such as dimethylformamide, dichloromethane or chloroform. The reaction is normally carried out at a non-extreme temperature for example - 30°C to + 50°C, preferably 0°C to + 30°C, and most conveniently at ambient temperature. The reaction is normally conducted in the presence of a base such as potassium, calcium, sodium, magnesium and lithium, carbonate or bicarbonate, preferably such bases being in an anhydrous form. Organic bases may be suitable also, for example triethylamine.

Suitable methods of hydrogenolysis for the preparation of compounds of the formula (I) include hydrogenation in the presence of a transition metal catalyst. The pressure of hydrogen used in the hydrogenation step may be low, medium or high, but in general an approximately atmospheric or slightly superatmospheric pressure is preferred. The transition metal catalyst employed is preferably palladium for example palladium on charcoal, palladium on barium sulphate or palladium on calcium carbonate. The hydrogenation may be effected in any convenient solvent in which the compound is soluble such as tetrahydrofuran, dioxan, ethanol, or such solvents in mixture with water. Phosphate buffer may also be included in the hydrogenation medium, this is particularly advantageous if it is desired to produce the compound of the formula (I) in the form of a zwitterion. If the hydrogenation is carried out in the presence of a base then a salt of a compound of the formula (I) is produced. Suitable bases include sodium bicarbonate, sodium carbonate, potassium carbonate, calcium carbonate and lithium carbonate. If no base is present then hydrogenation leads to the preparation of an acid of a compound of the formula (I) which may then may be neutralised if desired to yield a salt, such neutralisation being effected in conventional manner.

Re-esterification of a carboxyl group of a salt of a compound of the formula (I) may be effected in conventional manner. Suitable methods include the reaction of an alkali metal salt of the compound of the formula (I) such as the sodium or potassium salt with a reactive halide or sulphonate ester such as a bromide, chloride,

mesylate or tosylate. Such esterification may be carried out under conventional conditions for example in dimethylformamide at room temperature.

Compounds of the formula (II) may be prepared by the reaction of N-bromoacetamide with a corresponding compound of the formula (VII):

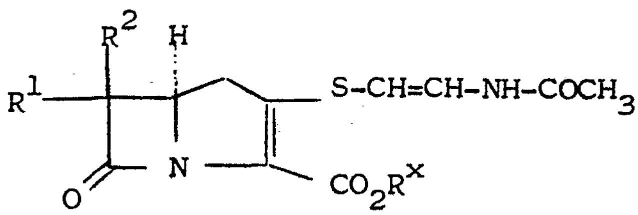

(VII)

wherein $R^1$, $R^2$ and $R^x$ are as defined in relation to formula (II). The reaction of the ester of the compound of the formula (VII) with N-bromoacetamide is most suitably carried out at a temperature such as - 15°C to + 25°C in a solvent such as moist acetone or dioxan. The desired product may be obtained by diluting with chloroform, washing with water and evaporating the dried organic layer.

The compounds of the formula (VII) and esters thereof may be obtainable directly from natural sources, or may be prepared by the modification of natural products. For example they may be prepared by the methods disclosed in UK Patent Specification Number 1489235, European Patent Application Publication Numbers 0004132, 0005349, 0007152 and 0005348, United States Serial Numbers 014,995 and 025,556 and Japanese Application Numbers 25014/79, 56215/79, 50233/79 and 56214/79, European Patent Application Publication Numbers 0001627 and 0001628 and OLS 2724560.

The groups $R^1$ and $R^2$ may be modified by such methods as are known for the modification of the C-6 substituent of known carbapenem derivatives. This may be performed after the formation of the S-$CH_2$-$CH_2$-$NHCOR^3$ side chain at the C-3 postion.

Certain compounds produced by the process of this invention are novel. Accordingly in a further aspect this invention provides a compound of formula (I) or a pharmaceutically acceptable salt or in-vivo hydrolysable ester thereof, wherein $R^1$ and $R^2$ are as defined with respect to formula (I), and $R^3$ is a group of formula $-NR^4R^5$ wherein $R^4$ and $R^5$ are as defined with respect to formula (I).

The present invention also provides a pharmaceutical composition which comprises a compound of the formula (I) or a pharmaceutically acceptable salt or in-vivo hydrolysable ester thereof wherein $R^1$ and $R^2$ are as defined hereinbefore and $R^3$ is a group $-NR^4R^5$ wherein $R^4$ and $R^5$ are as defined hereinbefore, and a pharmaceutically acceptable carrier.

Particularly suitable salts for use in such compositions include the sodium and potassium salts.

The compositions of this invention may be prepared by the conventional methods of preparation of antibiotic compositions and may be adapted for oral, topical or parenteral administration in conventional manner.

Aptly the compositions of this invention are in the form of a unit-dose composition adapted for oral administration.

Alternatively the compositions of this invention are in the form of a unit dose composition adapted for administration by injection.

Unit-dose forms according to this invention will normally contain from 50 to 500 mgs of a compound of this invention, for example about 62.5, 100, 125, 150, 200, 250 or 300 mgs. Such compositions may be administered from 1 to 6 times a day or more conveniently 2, 3 or 4 times a day so that the total daily dose for a 70 kg adult is about 200 to 2000 mg, for example about 400, 600, 750, 1000 or 1500 mg.

The compositions of this invention may be used to treat infections of the respiratory tract, urinary tract or soft tissues in humans, or may be used to treat mastitis in cattle.

The carriers used in the compositions of this invention may include diluents, binders, disintegrants, lubricants, colours, flavouring agents and preservatives in conventional manner. Thus suitable agents include lactose, starch, sucrose, calcium phosphate, sorbitol, polyvinylpyrrolidone, acacia, gelatin, tragacanth, potato starch, polyvinylpolypyrrolidone, magnesium stearate and sodium lauryl sulphate.

Orally administrable forms of the compositions of this invention are most suitably in the form of unit-dose units such as tablets or capsules.

In addition to being antibacterial agents in their own right the compounds of this invention are also able to enhance the effectiveness of penicillins and cephalosporins against β-lactamase producing strains of gram-positive and gram-negative bacteria presumably by virtue of being β-lactamase inhibitors.

The present invention also provides a synergistic pharmaceutical compostion which comprises a pharmaceutical composition as hereinbefore described which also contains a penicillin or a cephalosporin.

Suitable penicillins for inclusion in the compositions of this invention include benzyl penicillin, phenoxymethylpenicillin, ampicillin or a pro-drug therefor, amoxycillin or a pro-drug therefor, carbenicillin or a pro-drug therefor, ticarcillin or a pro-drug therefor, suncillin, sulbenicillin, azlocillin and mezlocillin.

Particularly suitable penicillins for inclusion in orally administrable compositions of this invention include ampicillin and its orally administrable pro-drugs, amoxycillin and its orally administrable pro-drugs and orally administrable pro-drugs of carbenicillin. Thus particularly suitable penicillins include ampicillin anhydrate, ampicillin trihydrate, sodium ampicillin, talampicillin hydrochloride, pivampicillin hydrochloride, bacampicillin hydrochloride, amoxycillin trihydrate, sodium amoxycillin; and the sodium salts of the phenyl and 5-indanyl α-esters of carbenicillin.

A preferred penicillin for inclusion in the orally administrable compositions of this invention is amoxycillin trihydrate. A further preferred penicillin for inclusion in the orally administrable compositions of this invention is ampicillin trihydrate.

Particularly suitable penicillins for inclusion in injectably administrable compostions of this invention include injectable salts such as the sodium salts of ampicillin, amoxycillin, carbenicillin and ticarcillin.

A preferred penicillin for inclusion in the injectably administrable compositions of this invention is sodium amoxycillin. A further preferred penicillin for inclusion in the injectably administrable compositions of this invention is sodium ampicillin.

Particularly suitable cephalosporins for inclusion in the compositions of this invention include cephaloridine, cephalexin, cephradine, cefazolin and cephalothin.

A particularly suitable cephalosporin for inclusion in the orally administrable compositions of this invention is cephalexin.

Particularly suitable cephalosporins for inclusion in the injectably administrable compositions of this invention include cefazolin and cephradine, generally as a pharmaceutically acceptable salt such as the sodium salt.

The weight ratio between a compound of this invention and a penicillin or cephalosporin is generally from 10:1 to 1:10, more usually from 5:1 to 1:5 and normally 3:1 to 1:3. The penicillin or cephalosporin is generally utilised in its conventionally administered amount.

The following Examples serve to illustrate the invention.

Example 1

p-Nitrobenzyl (5R,6S)-3-(2-acetamidoethylthio)-6-[(S)-1-hydroxyethyl]-7-oxo-1-azabicyclo[3.2.0]hept-2-ene-2-carboxylate.

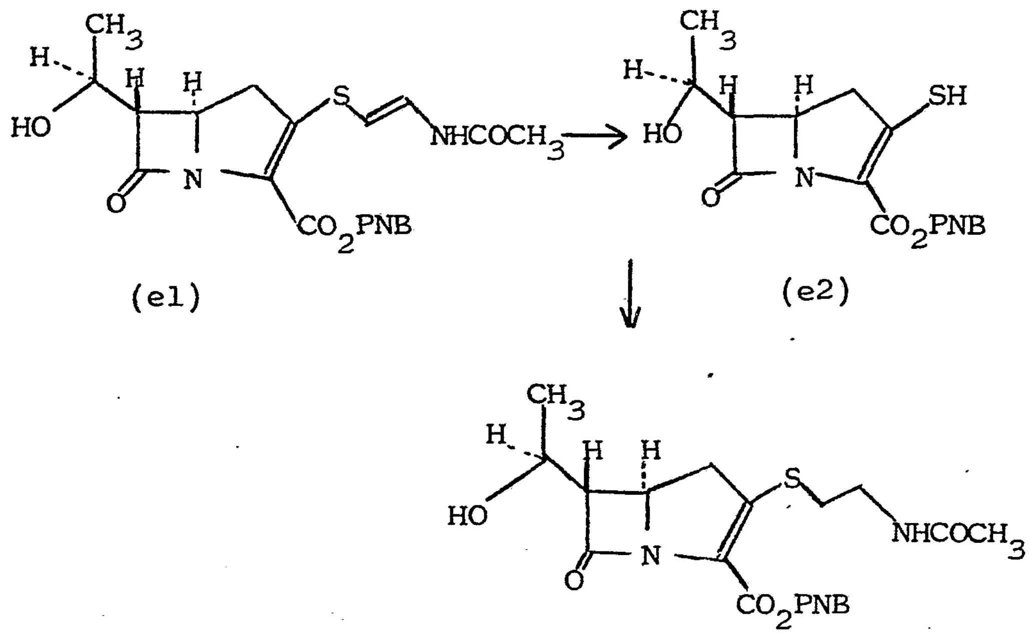

(e1) (e2)

(e3)

Step A

A solution of the ester (e1) (350 mg) in a mixture of dioxan (7 ml) and water (1 ml) was stirred with N-bromoacetamide (108 mg) at ambient temperature for 4.5 min. Chloroform (30 ml) was then added and the solution was washed with pH7 phosphate buffer (0.05M, 30 ml) and brine (30 ml). Evaporation of the dried ($MgSO_4$) organic layer afforded a foamy product which contained the thiol (e2).

Step B

Half of the product from step A was dissolved in DMF (1 ml) and stirred with anhydrous $K_2CO_3$ (11 mg)

for 20 min at room temperature in the presence of N-acetylaziridine (0.2 ml). Ethyl acetate (30 ml) was added and the organic solution was washed with water (4 x 30 ml) and brine (30 ml). The organic layer was dried ($MgSO_4$) and concentrated in vacuo to afford a crude product which was chromatographed on silica gel using a gradient elution of chloroform to 25% ethanol in chloroform. The major product was the title compound (e3) (20 mg).

Example 2

p-Nitrobenzyl (5R, 6S)-3-[2-(thiophen-2-carboxamido) ethylthio]-6-[(S)-1-hydroxyethyl]-7-oxo-1-azabicyclo [3.2.0]hept-2-ene-2-carboxylate

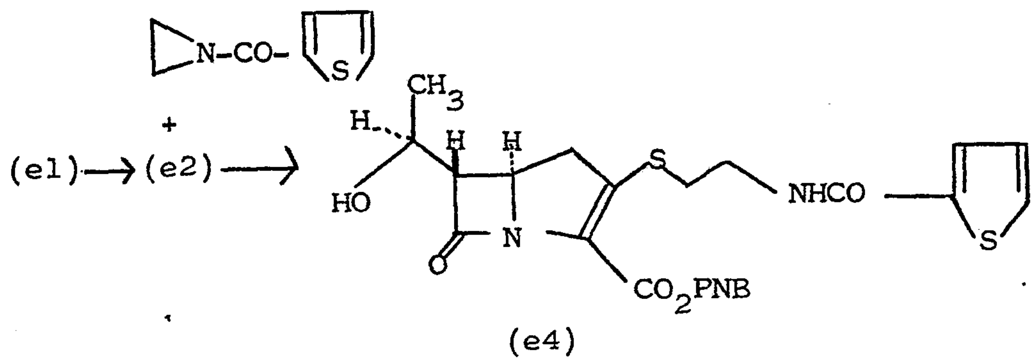
(e1) (e2)

(e4)

Step A

The ester (e1) (250 mg) was converted into the thiol (e2) by the method described in Example 1, step A.

Step B

The product from step A was dissolved in DMF (2 ml) and the solution was treated with potassium carbonate (14 mg) and N-ethylenethiophen-2-carboxamide (167 mg) with stirring at room temperature. After 20 min, ethyl acetate (30 ml) was added and the solution was washed with water (3 x 30 ml) and brine (30 ml). The organic phase was dried ($MgSO_4$) and concentrated in vacuo to yield a product which was chromatographed on silica gel using a gradient elution from 20% petroleum ether in ethyl acetate to 10% ethanol in ethyl acetate. The requisite fractions were collected and evaporated in vacuo to afford the title compound (e4) (127 mg); λ max (EtOH) 319 (12,759) and 266 (18,350) nm; ν max (KBr) 1770, 1695 and 1630 $cm^{-1}$; δ (DMF-d7) 1.27 (3H, d, J 6 Hz, $C\underline{H}_3CH$), 3.0 - 3.7 (7H, m, 4-$CH_2$, $SCH_2CH_2N$ and 6-CH), ca 4.0 - 4.4 (2H, m, 5-CH and $C\underline{H}CH_3$), 5.13 (1H, d, J 5 Hz, OH), 5.28 and 5.56 (each 1H, d, J 14 Hz, $C\underline{H}_2Ar$), 7.14 (1H, dd, J 3.5 and 5Hz, thiophen β-CH), 7.8 (4H, m, 2 x thiophen C$\underline{H}$ and 2 x $\underline{Ar}$), 8.25 (2H, m, 2 x $\underline{Ar}$), and 8.81 (1H, br, NH).

Example 3

p-Nitrobenzyl (5R,6R)-3-[2-(thiophen-2-carboxamido) ethylthio]-6-[(S)-1-hydroxyethyl]-7-oxo-1-azabicyclo [3.2.0.]hept-2-ene-2-carboxylate

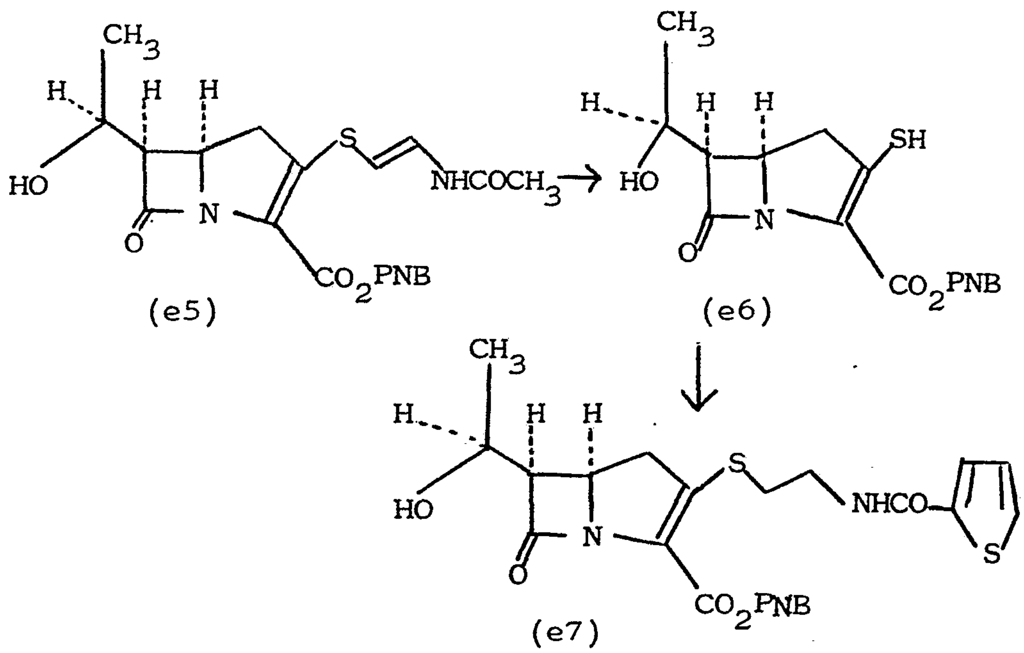

(e5) (e6) (e7)

Step A

The ester (e5) (400 mg) was converted to the thiol (e6) with N-bromoacetamide (123 mg) in the manner described in Example 1, step A.

Step B

The product from step A was treated with 1-(2-thenoyl)aziridine (267 mg) and potassium carbonate (22 mg) exactly as described in Example 2, step B. The title compound (e7) was obtained as a foam (172 mg) : λ max (EtOH) 318 and 266 nm; ν max ($CH_2Cl_2$) 3600, 3450, 1780, 1705 and 1650 $cm^{-1}$; δ(DMF-$d_7$) 1.30 (3H, d, J 6Hz, $C\underline{H}_3$-CH), 3.10-3.75 (7H, m, 4-$CH_2$,$SCH_2CH_2N$ and 6-CH), 3.95 - 4.45 (2H, m, 5-CH and $C\underline{H}CH_3$), 5.07 (1H, d, J 5.5 Hz, OH), 5.31 and 5.66 (each 1H, d, J 14 Hz, $C\underline{H}_2Ar$), 7.13 (1H, dd, J 3.5 and 5Hz, thiophen-β-CH), 7.80 (4H, m, 2 x

thiophen-CH and 2 x Ar), 8.26 (2H, d, J 9 Hz, 2 x Ar) and 8.82 (1H, br, NH).

Example 4

Sodium (5R,6R)-3-[2-(thiophen-2-carboxamido)ethylthio]-6-[(S)-1-hydroxyethyl]-7-oxo-1-azabicyclo[3.2.0] hept-2-ene-2-carboxylate

(e7)
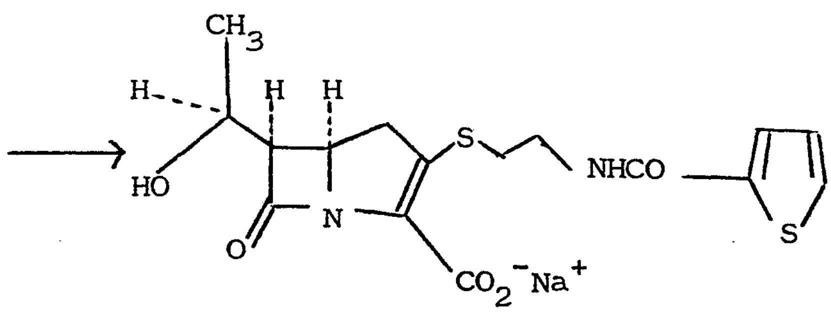

(e8)

A solution of the ester (e7) (120 mg) in 30% aqueous dioxan (10 ml) was shaken with hydrogen over 5% Pd on C catalyst (150 mg) which had been prehydrogenated in aqueous dioxan (5 ml) for 0.5h. After 3.25h the mixture was filtered over Celite washing with water (30 ml) and the filtrate was then concentrated in vacuo to a volume of ca 20 ml. The aqueous solution was washed with ethyl acetate (3 x 30 ml) and then further concentrated to ca 3 ml before loading onto a column (20 x 2.5 cm) of Biogel P2. Elution with water gave fractions containing the title compound (e8). These were combined and freeze-dried to afford the product as an amorphous solid (40 mg): λ max ($H_2O$) 300 sh, 275 and 255 nm.; ν max (KBr) 1750 and 1640-1590 $cm^{-1}$.

Example 5

The sodium salt of p-Nitrobenzyl (5R,6R)-3-[2-(thiophen-2-carboxamido)ethylthio]-6-[(S)-1-hydroxysulphonyloxyethyl]-7-oxo-1-azabicyclo [3.2.0]hept-2-ene-2-carboxylate

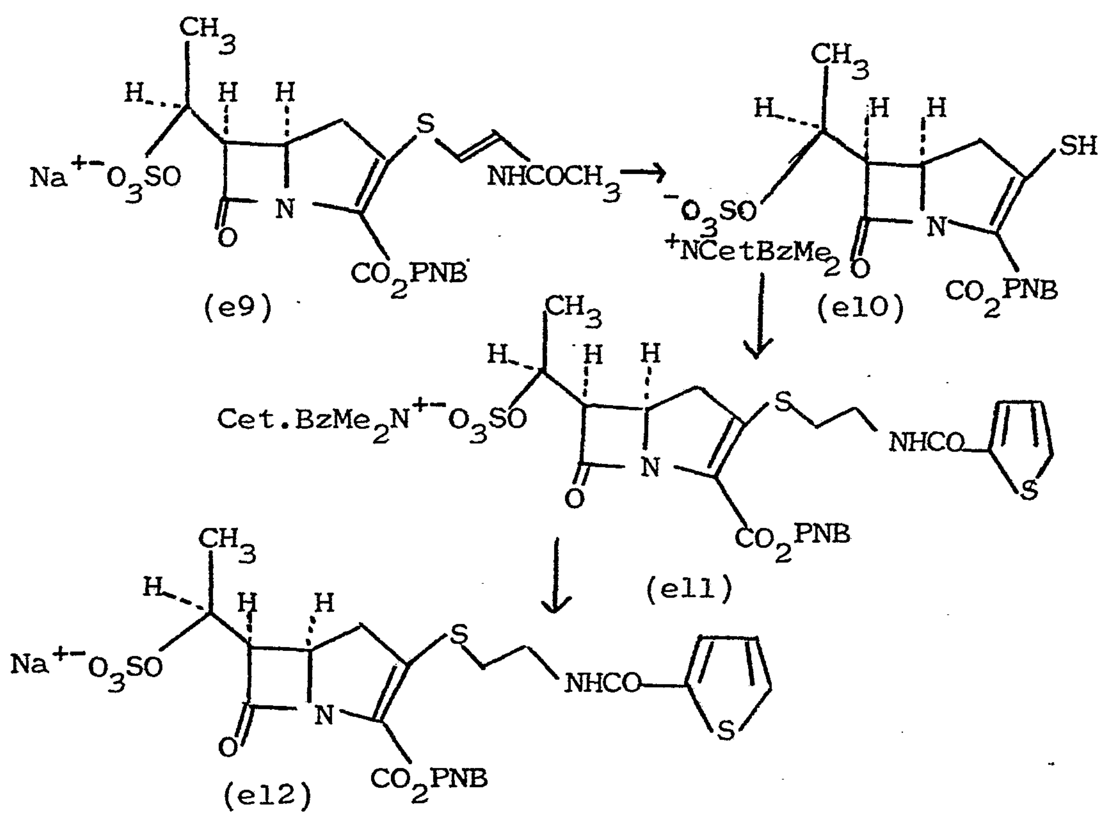

(e9) (e10) (e11) (e12)

Step A

A solution of the monoester; mono sodium salt (e9) (2.26 g) in water (50 ml) was thoroughly shaken with a solution of cetylbenzyldimethylammonium chloride (1.54 g) in dichloromethane (50 ml). The organic layer was separated, dried ($MgSO_4$) and evaporated in vacuo to afford a foam. This product was dissolved in a mixture of dioxan (20 ml) and water (3 ml) and the solution was then treated with N-bromoacetamide (482 mg) with stirring

at room temperature for 4.5 min. Chloroform (60 ml) was added and the solution was washed with water (30 ml) and brine (30 ml). Evaporation of the dried ($MgSO_4$) organic layer furnished a foamy product which contained the thiol (e10).

Step B

The product from step A was dissolved in DMF (12 ml) and the solution was stirred with 1-(2-thenoylaziridine) (1.0 g) and anhydrous potassium carbonate (100 mg) for 25 min. Ethyl acetate (100 ml) was added and the solution was washed with water (3 x 70 ml) and brine (50 ml). The organic phase was then dried ($MgSO_4$) and concentrated in vacuo. The product was chromatographed on silica gel using a gradient elution of $CHCl_3$ to 20% EtOH in $CHCl_3$. The quaternaryammonium salt (e11) was obtained as a foam (1.26 g); λ max (EtOH) 317 and 265 nm; ν max ($CH_2Cl_2$) 1780, 1705 and 1645 $cm^{-1}$.

Step C

A solution of (e11) (1.26g) in acetone (8 ml) and a solution of sodium iodide (198 mg) in acetone (3 ml) were mixed together. The white precipitate formed was filtered off washing well with acetone and ether, and then dried to give a solid (578 mg) which consisted of the title compound (e12); λ max ($H_2O$) 314 (10,911) and 270 (16,757); ν max (KBr) 1765, 1690 and 1630 $cm^{-1}$; δ (DMF-$d_7$) 1.48 (3H, d, *J* 6 Hz, C$\underline{H}_3$CH), *ca* 3.0 - 4.0 (7H, m, 4-$CH_2$, $SCH_2CH_2N$ and 6-CH), 4.15 - 4.80 (2H, m, 5-CH and $CH_3$C$\underline{H}$), 5.31 and 5.54 (each 1H, d, *J* 14 Hz, C$\underline{H}_2$Ar), 7.14 (1H, dd, *J* 3.5 and 5 Hz, thiophen β-CH), 7.70 - 7.95 (4H, m, 2 x thiophen C$\underline{H}$ and 2 x Ar), 8.27 (2H, d, *J* 8.5 Hz, 2 x Ar) and 8.83 (1H, br, NH).

0044142



Example 6

The disodium salt of (5R,6R)-3-[2-(thiophen-2-carboxamido)-ethylthio]-6-[(S)-1-hydroxysulphonyloxyethyl]-7-oxo-1-azabicyclo [3.2.0]hept-2-ene-2-carboxylic acid

(e12) →

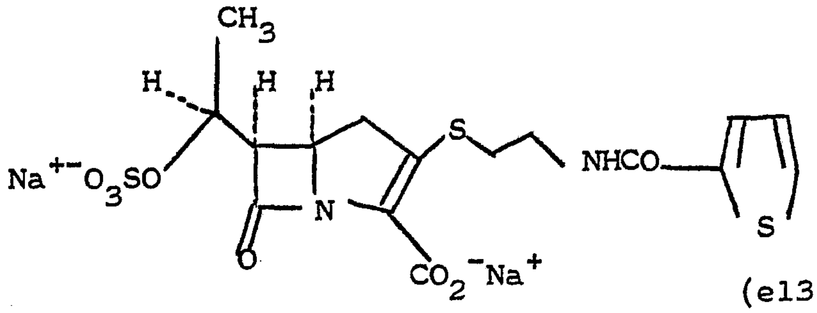

(e13)

A mixture of 5% Pd on C (150 mg) and 30% aqueous dioxan (10ml) was shaken under an atmosphere of hydrogen for 0.5 h. A solution of the ester (e12) (105 mg) in 30% aqueous dioxan (2 ml) was added to the mixture and hydrogenation was continued for a further 3.25 h. Work up and chromatography on Biogel P2 as described in Example 4 afforded fractions containing the title compound (e13). These were combined and freeze-dried to give the product as a solid (33 mg) : λ max ($H_2O$) 298 sh and 273 nm; ν max (KBr) 1755 and 1625 br $cm^{-1}$.

Example 7

p-Nitrobenzyl (5R, 6S)-3-[2-(p-nitrobenzyloxycarbonylamino-acetamido)ethylthio]-6-[(S)-1-hydroxyethyl]-7-oxo-1-azabicyclo[3.2.0]hept-2-ene-2-carboxylate

(e1) (e2)

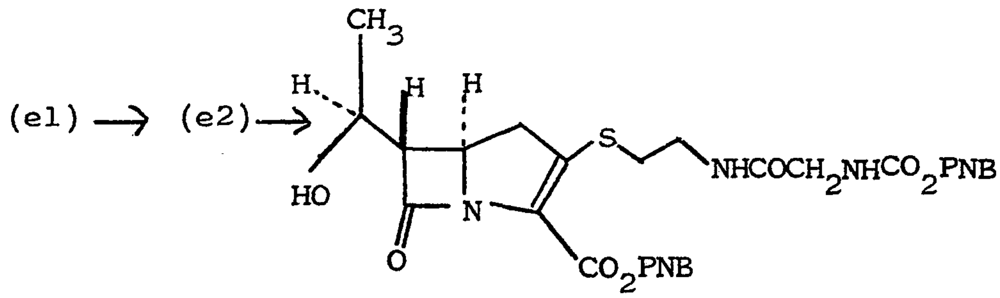

(e14)

Step A

The ester (e1) (500 mg) was treated with N-bromoacetamide (154 mg) as described in Example 1, step A, to afford the thiol (e2).

Step B

The thiol (e2) was treated with 1-(p-nitrobenzyloxycarbonylaminoacetyl) aziridine (312 mg) in the manner described in Example 2, step B. Chromatography on silica gel using a gradient elution of $CHCl_3$ to 25% $EtOH/CHCl_3$ gave the title compound (e14) as a yellow solid (107 mg): λ max (EtOH) 315 and 265 nm.; ν max (KBr) 1770, 1700 and 1675 sh $cm^{-1}$.

Example 8

(5R,6S)-3-(2-Aminoacetamidoethylthio)-6-[(S)-1-hydroxyethyl]-7-oxo-1-azabicyclo[3.2.0]hept-2-ene-2-carboxylic acid

(e14)

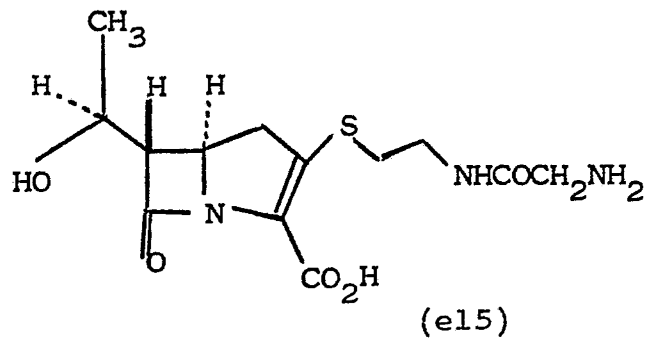

(e15)

A mixture of the ester (e14) (80 mg), 5% Pd on C (120 mg), dioxan (7 ml) and 0.05 M pH 7 phosphate buffer (3 ml) was shaken under an atmosphere of hydrogen for 2h. The mixture was filtered through Celite, washing with water and then concentrated to a volume of ca 20 ml. The aqueous solution was washed with ethyl acetate (5 x 30 ml) and further concentrated in vacuo (to 17 ml) to afford an aqueous solution of the title compound (e15) (23 mg): A portion of the solution was freeze-dried to give a solid; λ max ($H_2O$) 298 nm; ν max (KBr) 1750, 1665 and 1600 br $cm^{-1}$.

Example 9

p-Nitrobenzyl (5R,6R)-3-[2-(p-nitrobenzyloxycarbonyl-aminoacetamido)ethylthio]-6-[(S)-1-hydroxyethyl]-7-oxo-1-azabicyclo [3.2.0]hept-2-ene-2-carboxylate

(e5) (e6)

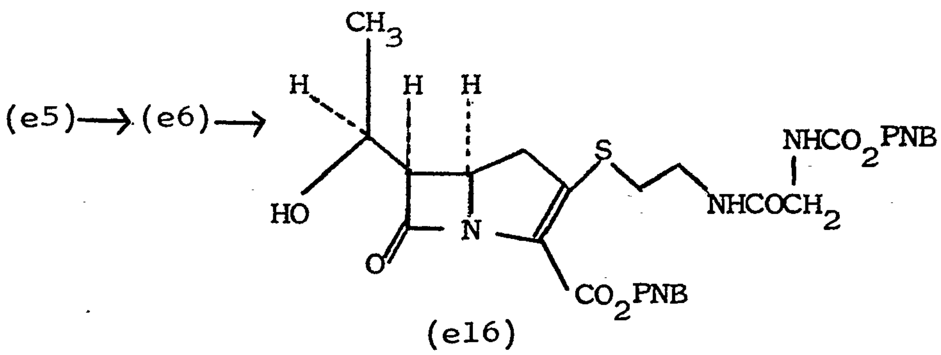

(e16)

Employing the methodology of Example 7, the ester (e5) (500 mg) was converted into the title compound (e16) (140 mg); λ max (EtOH) 314 (13000) and 266 (20,520) nm; ν max ($CH_2Cl_2$) 3430, 1780, 1730 and 1695 $cm^{-1}$.

Example 10

(5R,6R)-3-(2-Aminoacetamidoethylthio)-6-[(S)-1-hydroxyethyl]-7-oxo-1-azabicyclo[3.2.0]hept-2-ene-2-carboxylic acid

(e16)
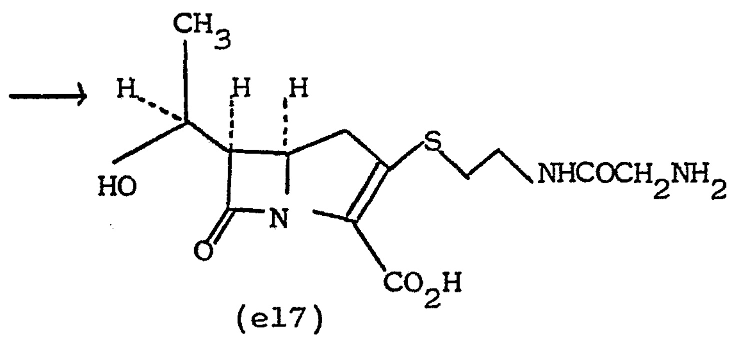
(e17)

Using the procedure of Example 8, the ester (e16) (55 mg) was hydrogenolysed to the title compound (e 17) (13 mg); λ max ($H_2O$) 298 nm.

Example 11

p-Nitrobenzyl (5R,6S)-3-(2-dimethylaminocarbonylaminoethylthio)-6-[(S)-1-hydroxyethyl]-7-oxo-1-azabicyclo [3.2.0]hept-2-ene-2-carboxylate

(e1) (e2)

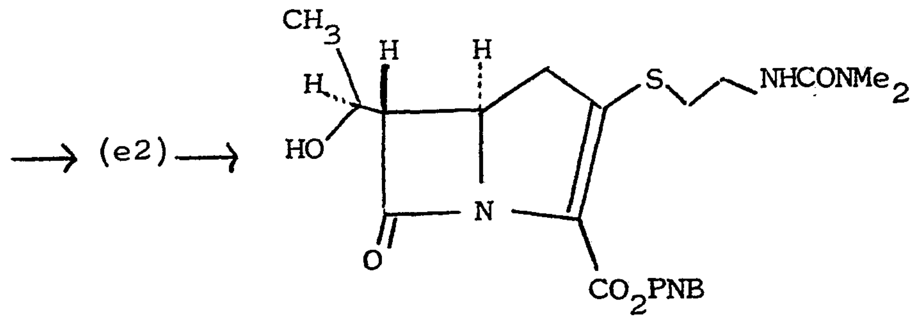

(e18)

Step A

The ester (e1) (500 mg) was converted into the thiol (e2) by the method of Example 1, step A.

Step B

The thiol (e2) was treated with N-(dimethylcarbamoyl)aziridine (255 mg) and $K_2CO_3$ (30 mg) as described in Example 1, step B. Chromatography of the product using $CHCl_3$ to 20% EtOH/$CHCl_3$ to elute afforded the title compound (e18) as a white solid (53 mg); λ max. (EtOH) 319 (11,275) and 265 (10,629) nm.; ν max. (KBr) 1770, 1695 and 1630 $cm^{-1}$; δ (DMF-$d_7$) 1.27 (3H, d, J 6Hz, $CH_3CH$), 2.82 (6H, s, $Me_2N$), ca. 2.9-3.6 (7H, m, 4-$CH_2$, $CH_2CH_2S$ and 6-CH), 3.95-4.35 (2H, m, 5-CH and $CH_3C\underline{H}$), 5.1 (1H, br, OH), 5.29 and 5.56 (each 1H, d, J 14Hz, $ArC\underline{H}_2$), 6.63 (1H, br, NH) and 7.80 and 8.24 (each 2H, d, J 9Hz, $\underline{Ar}CH_2$).

WHAT WE CLAIM IS:

1. A process for the preparation of a compound of the formula (I):

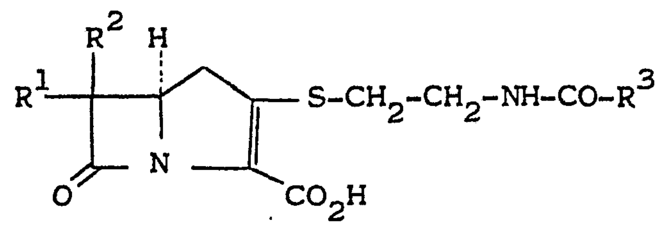

(I)

or a salt or ester thereof, wherein $R^1$ and $R^2$ each represent a hydrogen atom or an organic group bonded via a carbon atom to the carbapenem ring, and $R^3$ is a group $-NR^4R^5$ wherein $R^4$ is an optionally substituted $C_{1-6}$ alkyl group or an optionally substituted aryl or heteroaryl group or optionally substituted aryl $C_{1-6}$ alkyl or heteroaryl $C_{1-6}$ alkyl group and $R^5$ is a hydrogen atom or an optionally substituted $C_{1-6}$ alkyl group, or $R^3$ is an optionally substituted aryl or heteroaryl group or an optionally substituted $C_{1-6}$ alkyl group: which process comprises the reaction of a compound of the formula (II) or a reactive derivative thereof in which reaction any reactive groups in the groups $R^1$ and $R^2$ may be protected, if desired;

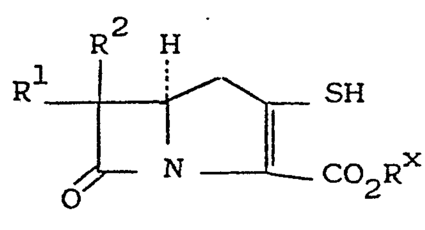

(II)

wherein $R^1$ and $R^2$ are as defined in relation to formula (I) and $R^x$ is a carboxy blocking group, with an aziridine derivative of the formula (III) in which any reactive groups in the group $R^3$ may be protected, if desired:

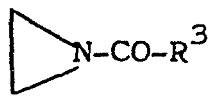

(III)

wherein $R^3$ is as defined in relation to formula (I); and thereafter optionally performing one or more of the following steps:

i) removing any protecting groups in the groups $R^1$, $R^2$ and $R^3$;

ii) removing any carboxy blocking group;

iii) converting the product into an ester, salt or acid.

2. A process as claimed in claim 1 for the preparation of a compound of the formula (IV):

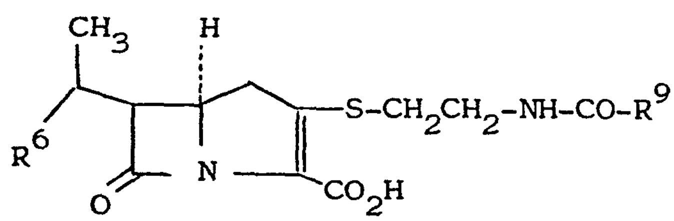

(IV)

or a pharmaceutically acceptable salt or in-vivo hydrolysable ester thereof wherein $R^6$ is hydrogen, OH, $OSO_3H$ or salt or methyl or ethyl ester thereof, and $R^9$ is a group $-NR^{10}R^{11}$ wherein $R^{10}$ is $C_{1-6}$ alkyl, phenyl or benzyl and $R^{11}$ is hydrogen or $C_{1-6}$ alkyl, or $R^9$ is an aryl or heteroaryl group or $C_{1-6}$ alkyl optionally substituted by aryl, heteroaryl, amino or by a carboxylic acid group or salt or $C_{1-4}$ alkyl ester thereof: with the proviso that when $R^6$ is $OSO_3H$ or a salt

or methyl or ethyl ester thereof the C-5 and C-6 protons are cis.

3. A process as claimed in either claim 1 or claim 2 wherein C-6 is substituted by an α-hydroxyethyl or α-sulphonato-oxyethyl group or salt or methyl or ethyl ester thereof.

4. A process as claimed in any of claims 1 to 3 wherein $COR^3$ is acetyl, propionyl, aminoacetyl, benzoyl, furoyl, thenoyl, methylaminocarbonyl or dimethylaminocarbonyl.

5. A process as claimed in claim 1 adapted to the preparation of a compound selected from: p-Nitrobenzyl (5R,6S)-3-(2-acetamidoethylthio)-6-[(S)-1-hydroxyethyl]-7-oxo-1-azabicyclo[3.2.0 ]hept-2-ene-2-carboxylate, p-Nitrobenzyl (5R,6S)-3-[2-(thiophen-2-carboxamido)ethylthio]-6-[(S)-1-hydroxyethyl]-7-oxo-1-azabicyclo[3.2.0] hept-2-ene-2-carboxylate, p-Nitrobenzyl (5R,6R)-3-[2-(thiophen-2-carboxamido)ethylthio]-6-[(S)-1-hydroxyethyl]-7-oxo-1-azabicyclo [3.2.0]hept-2-ene-2-carboxylate, Sodium (5R,6R)-3-[2-(thiophen-2-carboxamido)ethylthio] -6-[(S)-1-hydroxyethyl]-7-oxo-1-azabicyclo[3.2.0]hept-2-ene-2-carboxylate, The sodium salt of p-nitrobenzyl (5R,6R)-3-[2-(thiophen-2-carboxamido)ethylthio]-6-[(S)-1-hydroxysulphonyloxyethyl]-7-oxo-1-azabicyclo [3.2.0] hept-2-ene-2-carboxylate, The disodium salt of (5R,6R)-3-[2-(thiophen-2-carboxamido)ethylthio]-6-[(S)-1-hydroxysulphonyloxyethyl]-7-oxo-1-azabicyclo [3.2.0]hept-2-ene-2-carboxylic acid, p-Nitrobenzyl (5R,6S)-3-[2-(p-nitro-

benzyloxycarbonylamino-acetamido)ethylthio]-6-[(S)-1-hydroxyethyl]-7-oxo-1-azabicyclo [3.2.0]hept-2-ene-2-carboxylate, (5R,6S)-3-(2-Aminoacetamidoethylthio)-6-[(S)-1-hydroxyethyl]-7-oxo-1-azabicyclo[3.2.0]hept-2-ene-2-carboxylic acid, p-Nitrobenzyl (5R,6R)-3-[2-(p-nitrobenzyloxycarbonylaminoacetamido)ethylthio]-6-[(S)-1-hydroxyethyl]-7-oxo-1-azabicyclo [3.2.0]hept-2-ene-2-carboxylate, (5R,6R)-3-(2-Aminoacetamidoethylthio)-6-[(S)-1-hydroxyethyl]-7-oxo-1-azabicyclo[3.2.0]hept-2-ene-2-carboxylic acid and p-Nitrobenzyl (5R,6S)-3-(2-dimethylaminocarbonylaminoethylthio)-6-[(S)-1-hydroxyethyl]-7-oxo-1-azabicyclo[3.2.0]hept-2-ene-2-carboxylate.

6. A process as claimed in any of claims 1 to 4 when performed in the presence of a carbonate or bicarbonate of potassium, calcium, sodium, magnesium or lithium.

7. A compound of the formula (I) or a pharmaceutically acceptable salt or <u>in-vivo</u> hydrolysable ester thereof, wherein $R^1$ and $R^2$ are as defined in claim 1 and $R^3$ is a group $CONR^4R^5$ wherein $R^4$ and $R^5$ are as defined in claim 1.

8. A compound as claimed in claim 7 wherein one of $R^1$ and $R^2$ is a hydrogen atom and the other is a group of the sub-formula (i):

$$CH_3CHR^6- \qquad (i)$$

wherein $R^6$ is a hydrogen atom or OH, $OSO_3H$ or salt or

methyl or ethyl ester thereof, $OR^7$, $SR^8$, $OCO_2R^7$, $OCOR^8$ or $OCONHR^8$ wherein $R^7$ is $C_{1-4}$ alkyl, benzyl or nitrobenzyl and $R^8$ is $C_{1-4}$ alkyl, phenyl, benzyl or nitrobenzyl; $R^4$ is $C_{1-6}$ alkyl, phenyl or benzyl, and $R^5$ is hydrogen or $C_{1-6}$ alkyl.

9. A pharmaceutical composition comprising a compound as claimed in either claim 7 or 8 and a pharmaceutically acceptable carrier.

10. p-Nitrobenzyl (5R,6S)-3-(2-dimethylaminocarbonylaminoethylthio)-6-[(S)-1-hydroxyethyl]-7-oxo-1-azabicyclo [3.2.0]hept-2-ene-2-carboxylate.

European Patent Office

# EUROPEAN SEARCH REPORT

0044142

Application number

EP 81302738.0

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.3) |
|---|---|---|---|
| | GB - A - 1 531 141 (BEECHAM)<br>* Claims *<br>-- | 1-4 | C 07 D 487/04<br>A 61 K 31/40//<br>(C 07 D 487/04<br>C 07 D 205/00<br>C 07 D 209/00) |
| P | DE - A1 - 2 652 675 (MERCK)<br>* Claims, especially claim 6 *<br>& GB-A-1 570 986 (09-07-1980)<br>-- | 1-4, 7-9 | |
| P | DE - A1 - 2 652 680 (MERCK)<br>* Claims, especially claim 2 *<br>& GB-A-1 570 987 (09-07-1980)<br>---- | 1-4, 7-9 | |

TECHNICAL FIELDS SEARCHED (Int. Cl.3)

C 07 D 487/00
A 61 K 31/00

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons
&: member of the same patent family, corresponding document

X The present search report has been drawn up for all claims

| Place of search | Date of completion of the search | Examiner |
|---|---|---|
| VIENNA | 04-08-1981 | JANISCH |